# FASCICULE DE BREVET EUROPEEN

(11) **EP 3 302 644 B1**
(45) Date de publication et mention de la délivrance du brevet: **27.01.2021**
(21) Numéro de dépôt: 16736901.6
(22) Date de dépôt: 02.06.2016
(51) Int. Cl.: A61M 5/315, A61M 5/20, A61M 5/31, A61M 5/32

(54) **AUTOINJECTEUR.**
AUTOMATISCHER INJEKTOR
AUTO-INJECTOR

(30) Priorité: 05.06.2015 FR 1555166
(43) Date de publication de la demande: 11.04.2018
(73) Titulaire: Aptar France SAS, 27110 Le Neubourg (FR)
(72) Inventeur: FABIEN, David, 29810 Plouarzel (FR); GOMEZ, Thomas, 33160 Saint Aubin De Medoc (FR); PINHEIRO, Philippe, 27400 Incarville (FR); SAUSSAYE, Anthony, 61300 Saint Sulpice Sur Risle (FR)
(74) Mandataire: CAPRI
(86) Numéro de dépôt international: PCT/FR2016/051312
(87) Numéro de publication internationale: WO 2016/193622

(56) Documents cités:
- WO-A1-2013/078200
- WO-A1-2013/175140
- WO-A1-2013/175144
- WO-A2-2011/109205
- WO-A2-2012/022810

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois un certain nombre d'inconvénients.

Ainsi, notamment lorsque le volume de produit fluide est relativement important et/ou lorsque le produit fluide injecté est relativement visqueux, il est souhaitable de permettre au produit de diffuser du site d'injection pendant quelques secondes après ladite injection. Si l'utilisateur retire l'autoinjecteur immédiatement après la fin de l'injection, une partie du produit peut ressortir du corps de l'utilisateur ce qui diminue l'efficacité du traitement. Il est donc souhaitable de prévoir que l'utilisateur maintienne l'autoinjecteur contre son corps encore pendant quelques secondes après la fin de l'injection. Cet aspect est généralement résolu par les autoinjecteurs existants par la notice d'utilisation qui demande à l'utilisateur de compter dans sa tête un certain nombre de secondes avant de retirer le dispositif. Ceci n'est pas fiable et donc insatisfaisant, car le système dépend alors de l'utilisateur lui-même, qui dans certains cas peut être perturbé ou affaibli par l'action d'injection qu'il vient de réaliser.

Les documents WO2013175140, WO2013175144, WO2013078200, WO2012022810 et WO2011109205 décrivent des autoinjecteurs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui permet à l'utilisateur de déterminer quand il doit retirer ou quand il peut retirer l'autoinjecteur de son corps après son utilisation, qui soit sûr et qui empêche tout risque de blessure, et qui soit simple et peu coûteux à fabriquer et à assembler.

La présente invention a donc pour objet un autoinjecteur tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
Les figures 1a et 1b sont des vues schématiques, respectivement de côté et en section transversale, d'un autoinjecteur selon un premier mode de réalisation avantageux de la présente invention, en position après piquage et avant injection,
Les figures 2a et 2b sont des vues similaires à celles des figures 1a et 1b, après injection et en cours d'actionnement du dispositif indicateur,
Les figures 3a et 3b sont des vues similaires à celles des figures 2a et 2b, en fin d'actionnement du dispositif indicateur,
La figure 4 est une vue similaire à celle de la figure 3a, en position de fin d'utilisation, après retrait de l'autoinjecteur du site d'injection,
La figure 5 est une vue en perspective éclatée d'une partie de l'autoinjecteur selon ledit premier mode de réalisation des figures 1 à 4,
La figure 6 est une vue en perspective éclatée du dispositif indicateur selon ledit premier mode de réalisation des figures 1 à 5,
Les figures 7 et 8 sont des vues en perspective découpée du dispositif indicateur de la figure 6, respectivement avant et après actionnement du système retardateur,
Les figures 9 et 10 sont des vues en perspective de détail du fonctionnement du dispositif indicateur de la figure 6, vue d'en haut,
La figure 11 est une vue schématique de détail en section transversale d'une partie de l'autoinjecteur des figures 1 à 5, montrant plus particulièrement le dispositif indicateur de la figure 6, dans la position de la figure 1b,
Les figures 12a à 12d sont des vues schématique en coupe transversale, respectivement selon les plans de coupe A-A, B-B, C-C et D-D de la figure 11,
Les figures 13a et 13b sont des vues schématiques, respectivement de côté et en section transversale, d'un autoinjecteur selon un second mode de réalisation avantageux de la présente invention, en position après injection et en début d'actionnement du système retardateur,
La figure 14 est une vue en perspective éclatée du système retardateur selon ledit second mode de réalisation des figures 13a et 13b,
La figure 15 est une vue schématique de détail en section transversale d'une partie de l'autoinjecteur des figures 13 et 14, montrant plus particulièrement le système retardateur,
Les figures 16a à 16d sont des vues schématique en coupe transversale, respectivement selon les plans de coupe A-A, B-B, C-C et D-D de la figure 15,
La figure 17 est une vue schématique en coupe transversale selon le plan de coupe E-E de la figure 15, illustrant une variante de réalisation avantageuse,
Les figures 18a et 18b sont des vues de détails en perspective d'une autre variante de réalisation,
La figure 19 est une vue de détail en perspective d'encore une autre variante de réalisation,
Les figures 20a et 20b sont des vues schématiques, respectivement de côté et en section transversale, d'un autoinjecteur selon un troisième mode de réalisation avantageux de la présente invention, en position après injection et en début d'actionnement du système retardateur,
La figure 21 est une vue en perspective éclatée du système retardateur selon ledit second mode de réalisation des figures 20a et 20b,
La figure 22 est une vue schématique de détail en section transversale d'une partie de l'autoinjecteur des figures 20 et 21, montrant plus particulièrement le système retardateur, et
Les figures 23a à 23c sont des vues schématique en coupe transversale, respectivement selon les plans de coupe A-A, B-B et C-C de la figure 22.

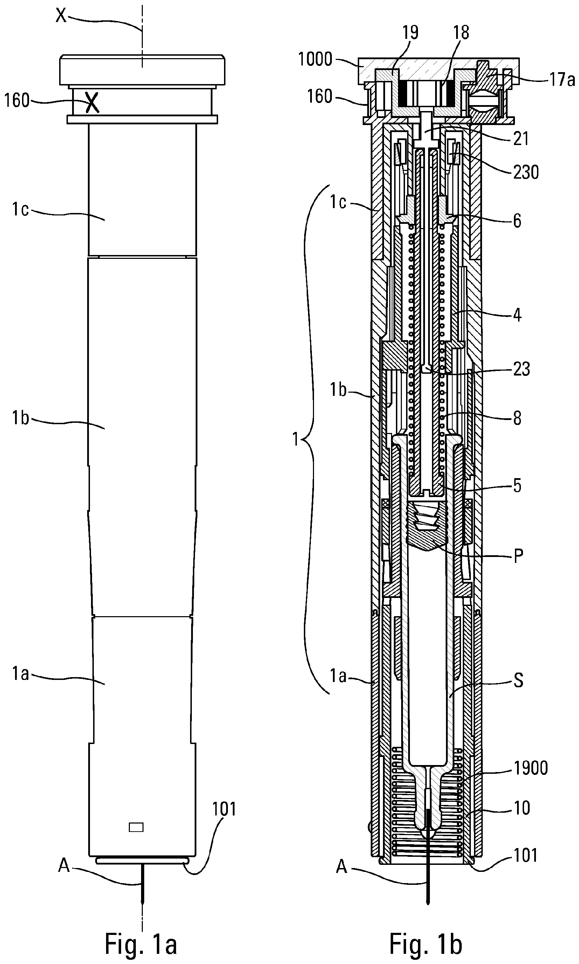

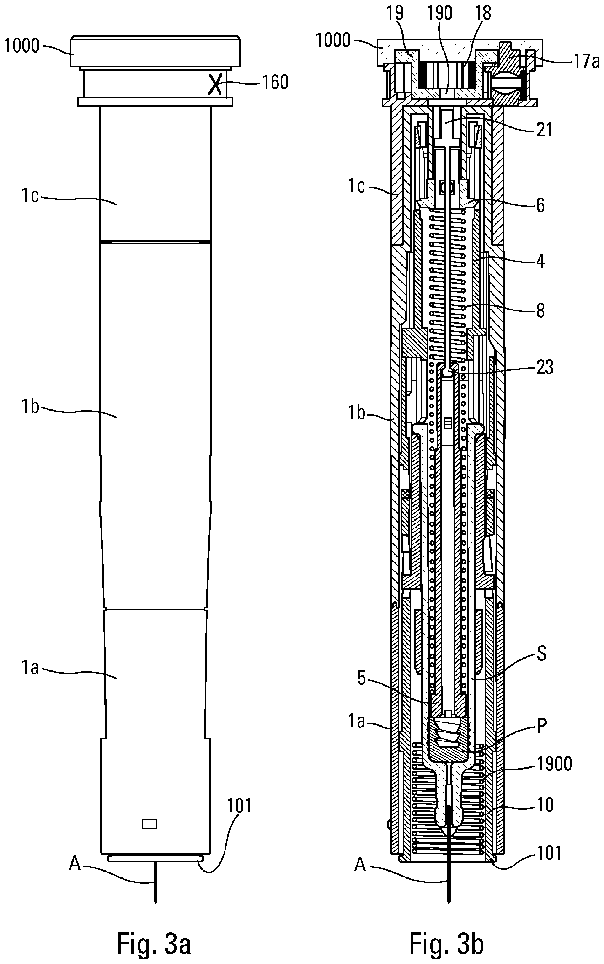

Dans la description ci-après, les termes "supérieur", "inférieur", "haut" et "bas" se réfèrent aux positions représentées sur les figures 1a à 4, 11, 13a, 13b, 15, 20a, 20b et 22. Les termes "axial" et "radial" se réfèrent à l'axe central longitudinal X, représenté notamment sur les figures 1a, 13a et 20a, qui correspond à l'axe longitudinal de l'aiguille.

L'autoinjecteur va être décrit ci-après en référence à trois modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

L'autoinjecteur représenté sur les figures comporte un corps 1 dans lequel coulisse axialement un manchon actionneur 10, dont l'extrémité inférieure 101 est destinée à venir en contact avec le corps du patient autour de la zone d'injection. Dans les exemples, l'autoinjecteur comporte un corps inférieur 1a, un corps intermédiaire 1b et un corps supérieur 1 c qui sont assemblés pour former le corps 1 de l'autoinjecteur. Ci-après, le terme "corps" et la référence numérique "1" seront utilisés pour désigner ledit corps unitaire formé par l'assemblage dudit corps inférieur 1a avec ledit corps intermédiaire 1b et ledit corps supérieur 1c. Il est à noter que le corps 1 pourrait être formé d'un nombre quelconques de parties de corps, et que les modes de réalisation des figures, avec trois parties de corps, ne sont pas limitatifs.

Un réservoir S peut être inséré dans ledit corps 1 de l'autoinjecteur, ledit réservoir S étant de préférence fixe dans ledit corps 1. Ce réservoir S contient du produit fluide, et comporte un piston P et une aiguille A. Le piston P est adapté à se déplacer dans ledit réservoir S pour injecter le produit fluide à travers ladite aiguille A. Eventuellement, la présente invention pourrait aussi s'appliquer à un réservoir sans aiguille, notamment dans un dispositif d'injection sans aiguille.

La présente description sera faite en référence à une seringue S, qui peut être de tout type. Plus généralement, il est entendu que le terme "seringue" dans la présente description englobe tout type de réservoir associé à une aiguille. De préférence, le réservoir S est une seringue préremplie.

Avant actionnement de l'autoinjecteur, l'aiguille A de la seringue S peut être protégée par un capuchon (non représenté), l'autoinjecteur pouvant comporter un capot (non représenté) que l'utilisateur peut retirer avant actionnement. Le retrait du capot provoque avantageusement le retrait du capuchon.

Avant actionnement, le manchon actionneur 10 est dans une première position projetée dans laquelle il entoure l'aiguille A.

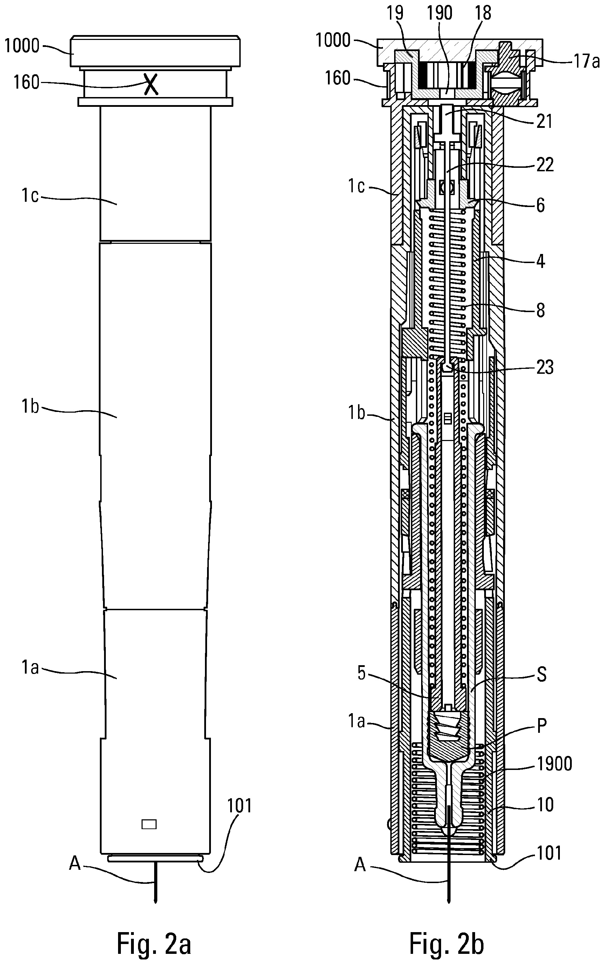

Lors de l'actionnement, le manchon actionneur 10 coulisse à l'intérieur du corps 1 vers une position d'actionnement, pour exposer l'aiguille A et permettre le piquage puis l'injection du produit fluide. La figure 1b illustre l'autoinjecteur après piquage mais avant injection. Les figures 2b, 3b, 13b et 20b illustrent l'autoinjecteur après injection.

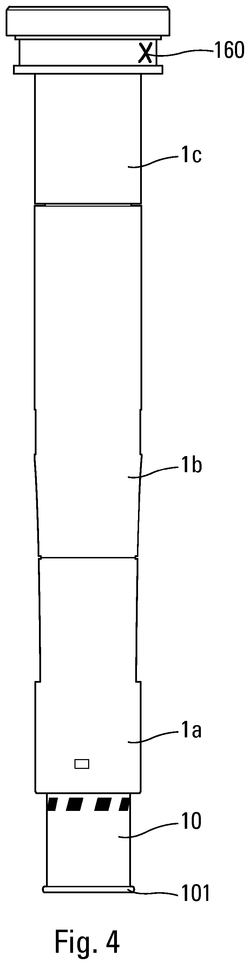

Après l'injection, lorsque l'utilisateur retire l'autoinjecteur du site d'injection, le manchon actionneur 10 revient dans une seconde position projetée de fin d'utilisation, dans laquelle il est à nouveau disposé autour de l'aiguille A, pour éviter tout risque de blessure avec ladite aiguille, comme représenté sur la figure 4.

Le manchon actionneur 10 est avantageusement sollicité vers ses positions projetées par un organe élastique ou ressort 190, qui peut être de type quelconque. Avantageusement, dans ladite position de fin d'utilisation, ledit manchon actionneur 10 est verrouillé, et ne peut plus être déplacé axialement vers l'intérieur dudit corps 1. Ce verrouillage peut par exemple être réalisé par des pattes (non représentées), solidaires du corps 1 ou du réservoir S, et coopérant avec des ouvertures (non représentées) dudit manchon actionneur 10 lorsque celui-ci atteint sa seconde position projetée. Ce verrouillage, qui n'est pas essentiel au fonctionnement de la présente invention, ne sera pas décrit plus en détail ci-après. Il pourrait être réalisé différemment du mode de réalisation particulier mentionné ci-dessus. En particulier, il pourrait être réalisé conformément aux enseignements des documents WO2013175140 ou WO2013175142.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée. Le ressort d'injection 8 n'est représenté que pour les premier et second modes de réalisation.

Une serrure d'injection avantageuse est notamment décrite dans le document WO2015155484.

La serrure peut comprendre au moins un élément de blocage (non représenté), retenu en position de blocage par une bague de blocage 230 fixée, notamment encliquetée sur un organe support 6 sur lequel s'appuie le ressort d'injection 8. Le déclenchement de ladite serrure d'injection provoque l'actionnement desdits moyens d'injection et donc l'injection du produit fluide à travers l'aiguille. Ladite serrure d'injection peut en outre comporter un manchon de commande 4 disposé dans ledit corps 1, ledit manchon de commande 4 contenant ladite tige de piston 5 et ledit ressort d'injection 8, ladite tige de piston 5 comportant un évidement radial recevant au moins un élément de blocage mobile entre une position de blocage et une position de déblocage. Ledit au moins un élément de blocage est de préférence de forme sensiblement sphérique, tel qu'une bille. Avantageusement, lesdites billes sont sollicitées radialement vers l'extérieur par ladite tige de piston 5 et sont retenues en position de blocage par la bague de blocage 230. Cette bague de blocage 230 est déplaçable axialement par rapport à ladite tige de piston 5 et par rapport audit organe support 6 entre une position de verrouillage, dans laquelle elle maintient lesdites billes en position de blocage, et une position de déverrouillage, dans laquelle lesdites billes sont libérés pour ainsi débloquer ladite serrure d'injection, permettant audit ressort d'injection 8 de déplacer ladite tige de piston 5 vers sa position d'injection. La bague de blocage 230 peut notamment être déplacée vers sa position de déverrouillage par ledit manchon de commande 4.

Lorsque l'aiguille A de la seringue S a pénétré le corps de l'utilisateur, la bague de blocage 230 est déplacée axialement vers le haut, ce qui a pour effet de libérer les billes de leur position de blocage, celles-ci étant alors déplacées radialement vers l'extérieur. La tige de piston 5 n'est alors plus retenue par les billes et elle est donc déplacée axialement vers le bas pour réaliser l'injection du produit.

L'autoinjecteur comporte un dispositif indicateur pour indiquer à l'utilisateur, notamment par un son audible, par une vibration et/ou par une indication visuelle et/ou tactile, qu'il peut retirer l'autoinjecteur du site d'injection. Ledit dispositif d'indication visuel, sonore et/ou tactile est disposé de préférence à l'extrémité arrière dudit corps 1, opposée audit site d'injection. En particulier, la présente invention prévoit de générer un bruit pendant l'actionnement du dispositif indicateur, la fin dudit bruit signifiant la fin d'actionnement du dispositif indicateur. Avantageusement dans les exemples représentés, le dispositif indicateur comporte en outre un élément indicateur qui donne une indication visuelle, par un affichage 160 adéquat dans une ou plusieurs fenêtre(s) 11 du corps 1. Avantageusement une indication tactile peut également être fournie.

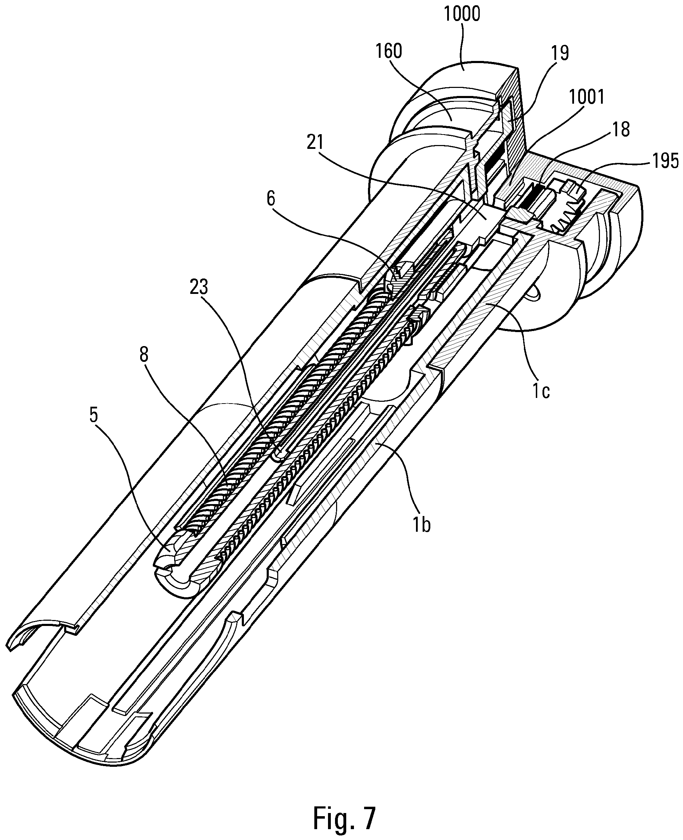

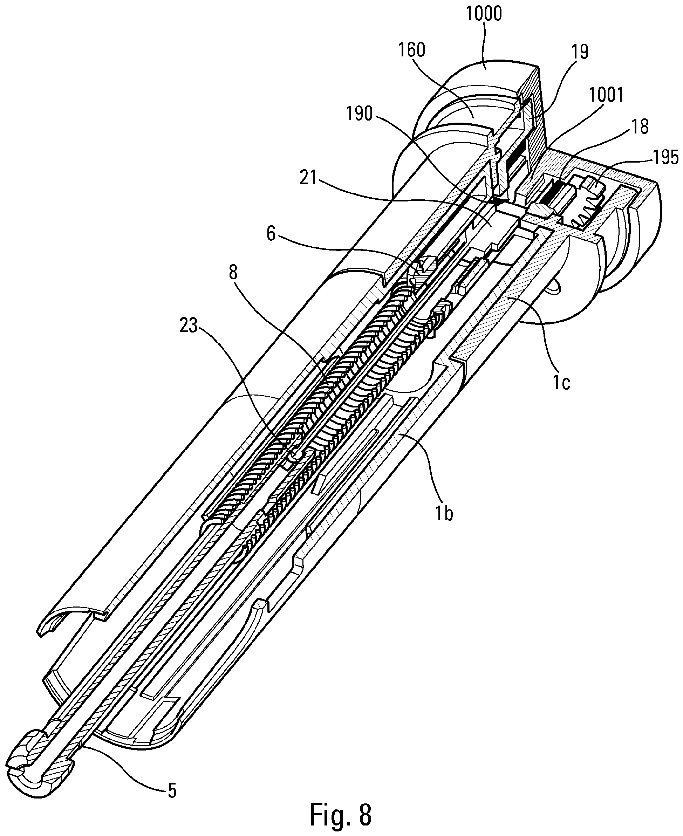

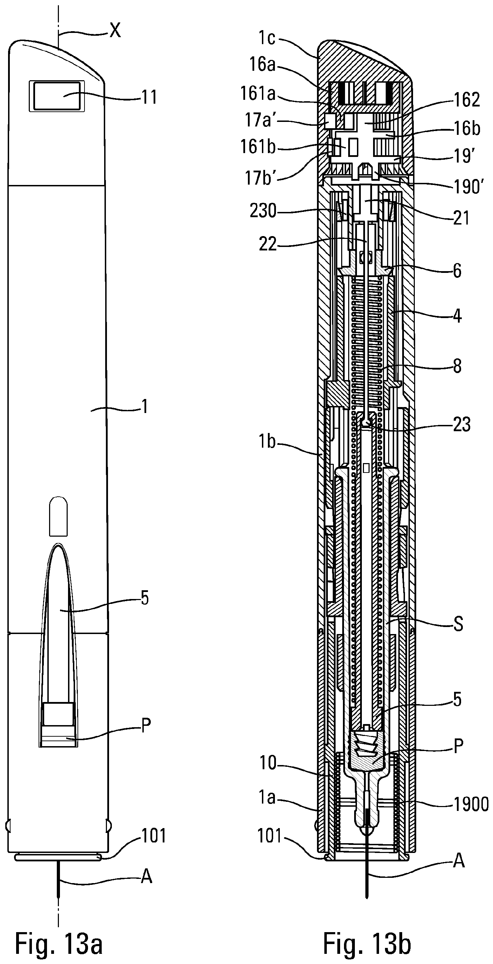

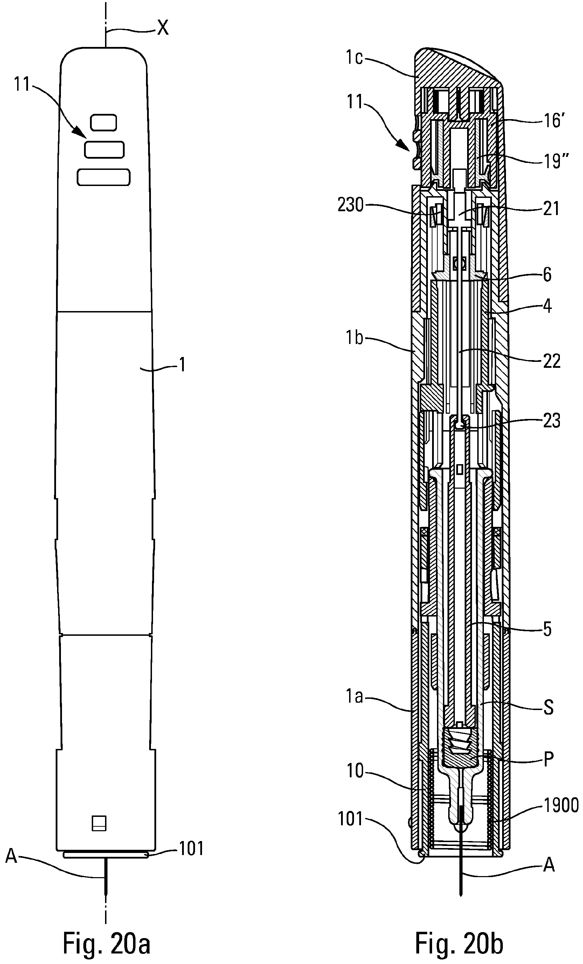

Les figures 1 à 12 illustrent un dispositif indicateur selon un premier mode de réalisation avantageux, les figures 13 à 19 illustrent un second mode de réalisation avantageux, et les figures 20 à 23 illustrent un troisième mode de réalisation avantageux.

Pour éviter que l'utilisateur ne retire l'autoinjecteur du site d'injection dès la fin d'injection, l'autoinjecteur comporte un système retardateur, qui va retarder la fin d'actionnement dudit dispositif indicateur par rapport à la fin de l'injection.

Le système indicateur a principalement pour but de générer une indication sonore pendant un temps prédéterminé après la fin de l'injection du produit fluide à l'intérieur dudit corps. Ce temps permet notamment une diffusion pendant quelques secondes du produit après son injection. Un tel indicateur procure aussi un bénéfice pour l'utilisateur, qui n'a plus à compter, par exemple jusqu'à 10, après son injection, le temps pris pour ce comptage pouvant être très variable d'un utilisateur à un autre. Avec un dispositif indicateur associé à un système retardateur, on facilite la séquence d'utilisation d'un autoinjecteur.

Avantageusement, le dispositif indicateur et/ou le système retardateur comporte un élément mobile en rotation par rapport au corps, le son continu étant généré pendant cette rotation, par exemple au moyen d'une ou plusieurs pattes flexibles formées soit sur ledit élément mobile, soit sur ledit corps, et coopérant avec des profils adaptés formés sur l'autre parmi ledit élément mobile et ledit corps.

Avantageusement, on peut envisager de moduler le son produit par lesdites pattes sur lesdits profils adaptés, par exemple :
- en ajustant les formes et/ou les dimensions des profils, et/ou
- en utilisant au moins deux pattes ayant des géométries différentes. Ainsi, le son produit pourrait par exemple :
- aller d'un son grave au début à plus à un son aiguë à la fin, ou inversement, et/ou
- être multi-tons, et/ou
- avoir une intensité qui évolue pendant le retardement.

Le premier mode de réalisation des figures 1 à 12 utilise une bande souple du type cassette VHS pour générer une indication sonore continue, ainsi qu'avantageusement une indication visuelle correspondante. En variante, on pourrait remplacer la bande souple par un fil ou similaire.

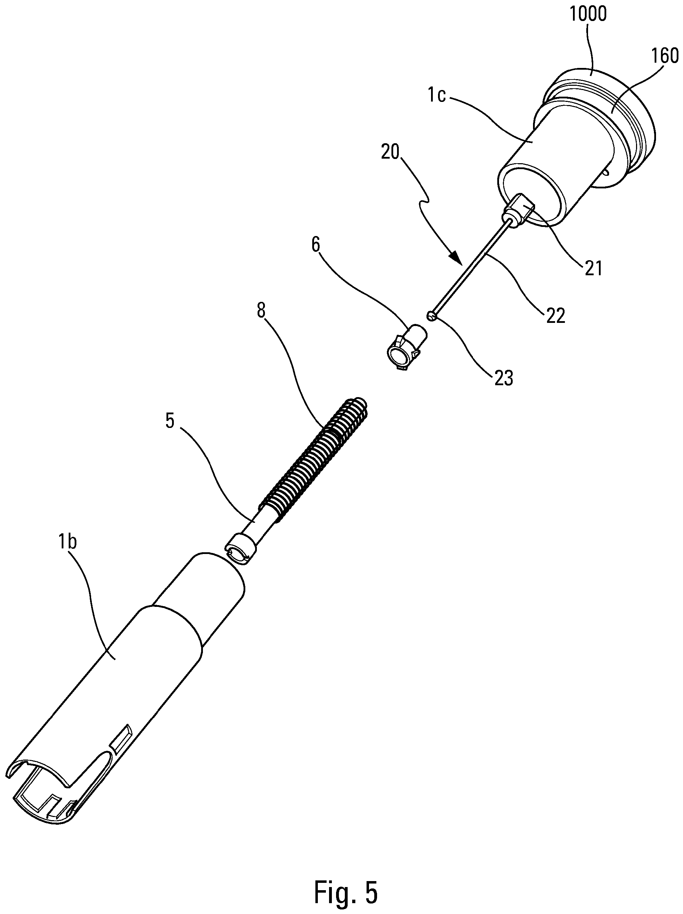

La figure 5 illustre une vue schématique en perspective éclatée de la partie de l'autoinjecteur incorporant ce système indicateur selon le premier mode de réalisation. Celui-ci comprend un module indicateur, une clé de verrouillage 20, le ressort d'injection 8, la tige de piston 5, l'organe support 6 et le corps intermédiaire 1b.

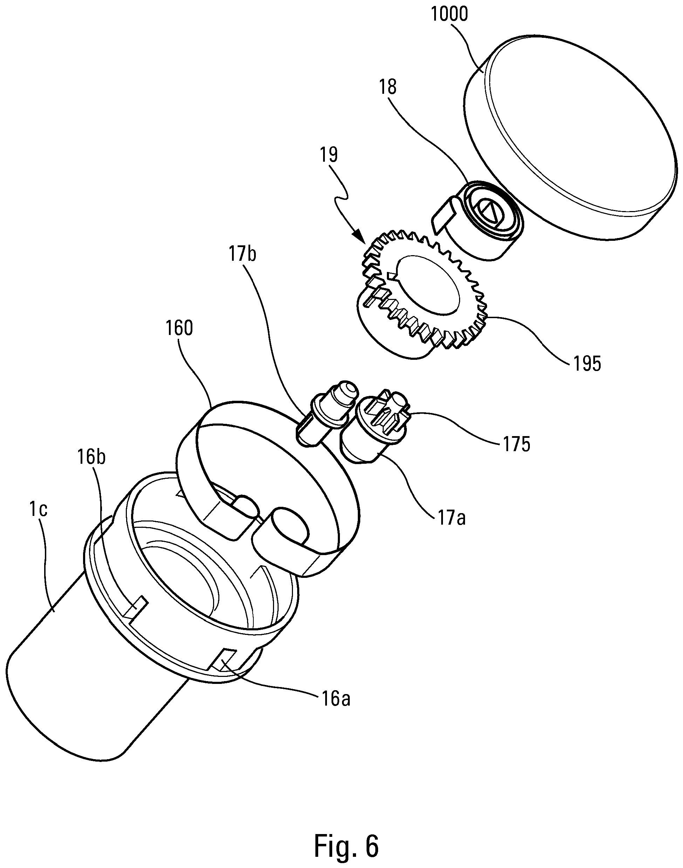

La figure 6 illustre une vue schématique en perspective éclatée du module indicateur de la figure 5. Celui-ci comprend un capot 1000, le corps supérieur 1c, une bande indicatrice 160, un ressort indicateur 18, de préférence réalisé sous la forme d'un ressort à spirale, une roue motrice 19, une roue d'enroulement 17a et une roue de déroulement 17b.

La roue motrice 19 comporte un engrenage 195 qui coopère avec un engrenage 175 de la roue d'enroulement 17a. La roue de déroulement 17b est avantageusement librement rotative sous l'effet de la traction exercée sur elle par la bande indicatrice 160 lorsque celle-ci s'enroule sur ladite roue d'enroulement 17a.

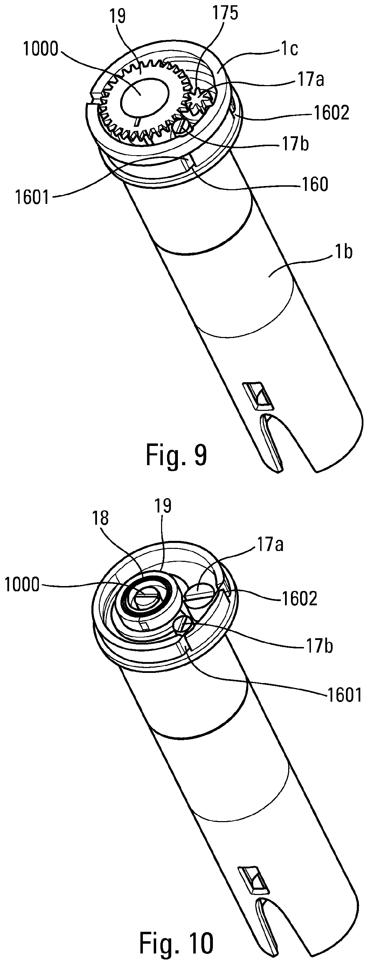

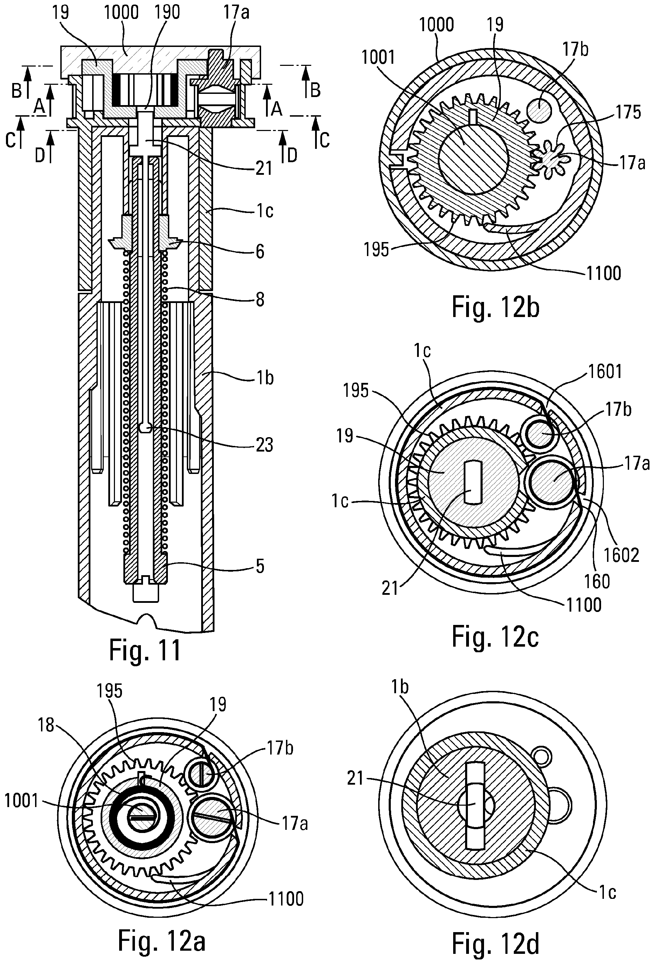

Le ressort à spirale 18 est fixé d'une part au capot 1000, notamment sur une projection axiale centrale 1001 de celui-ci, et d'autre part à la roue motrice 19, comme visible notamment dans les figures 10 et 12a.

La bande indicatrice 160 est enroulée sur ladite roue de déroulement 17b, puis s'étend à travers une première fente 1601 du corps supérieur 1c pour faire le tour dudit corps supérieur 1c et passer par une seconde fente 1602 pour enfin s'enrouler sur ladite roue d'enroulement 17a.

La clé de verrouillage 20 comporte une tête 21 adaptée à coopérer avec le module indicateur, une tige longitudinale 22, et en embout 23 adapté à coopérer avec la tige de piston 5.

En position avant déclenchement du système indicateur, la tête 21 de la clé de verrouillage 20 est en position de blocage dans laquelle elle coopère avec un profil correspondant 190 de la roue motrice 19, de sorte que celle-ci est bloquée en rotation par ladite clé. Lorsque la tige de piston 5 arrive vers sa position de fin d'injection, elle va coopérer avec l'embout 23 de la clé de verrouillage 20, et ainsi tirer ladite clé 20 axialement vers le bas. De ce fait, la tête 21 de ladite clé de verrouillage 20 sort axialement dudit profil 190 de la roue motrice 19, de sorte que ladite roue motrice 19 n'est plus bloquée en rotation par ladite clé 20.

Le ressort à spirale 18 sollicite la roue motrice 19 en rotation. Tant que la roue motrice 19 est bloquée par ladite clé de verrouillage 20, le dispositif indicateur est donc également bloqué.

Lorsque le dispositif indicateur est déclenché, le ressort 18 sollicite en rotation la roue motrice 19. Celle-ci entraine en rotation la roue d'enroulement 17a, qui va enrouler la bande 160, ce qui va simultanément la dérouler de la roue de déroulement 17b.

Pendant la rotation de la roue motrice 19, au moins une patte flexible 1100 du corps 1, notamment du corps supérieur 1c, coopère avec l'engrenage 195 de la roue motrice 19 pour générer un son continu. En variante, cette patte flexible 1100 pourrait aussi coopérer avec une autre partie de la roue motrice 19, ou la roue motrice 19 pourrait incorporer une ou plusieurs pattes flexibles coopérant avec des profils du corps 1. Lorsque la roue motrice 19 s'arrête de tourner, le son s'arrête aussi, et l'utilisateur sait qu'il peut retirer l'autoinjecteur du site d'injection.

Avantageusement, la bande 160 comporte des marquages, par exemple des zones de couleur et/ou des signes ou symboles, par exemples des chiffres, des lettres, ou tout autre type de marquage, pour informer visuellement l'utilisateur. Sur les figures 1a, 2a, 3a et 4a, ce marquage a été représenté par un X, mais ceci n'est qu'un exemple pour illustrer le déplacement de la bande 160 lors de l'actionnement du dispositif indicateur. Ces marquages sont visibles de l'utilisateur sur la partie externe du corps supérieur 1c sur laquelle ladite bande indicatrice 160 s'étend, entre lesdites première et seconde fentes 1601, 1602. Eventuellement, le corps supérieur pourrait en outre comporter une ou plusieurs fenêtres affichant par exemple la fin d'utilisation de manière visuelle en plus de l'information sonore fournie par la fin dudit bruit continu. En variante, la bande pourrait comporter un ou plusieurs trous ou découpes permettant de faire apparaitre un ou plusieurs marquages réalisés sur le corps 1, avant, pendant et/ou après enroulement de ladite bande.

La durée d'indication et/ou le retardement de la fin d'indication par rapport à la fin de l'injection peuvent donc être prédéterminés en ajustant les paramètres du dispositif indicateur, et notamment la longueur de la bande indicatrice 160, les dimensions des engrenages 195, 175 des roues motrice 19 et d'enroulement 17a, les caractéristiques du ressort indicateur 18, etc.

Le second mode de réalisation des figures 13 à 19 exploite un train épicycloïdal pour générer ledit retard entre la fin d'actionnement du dispositif indicateur et la fin de l'injection.

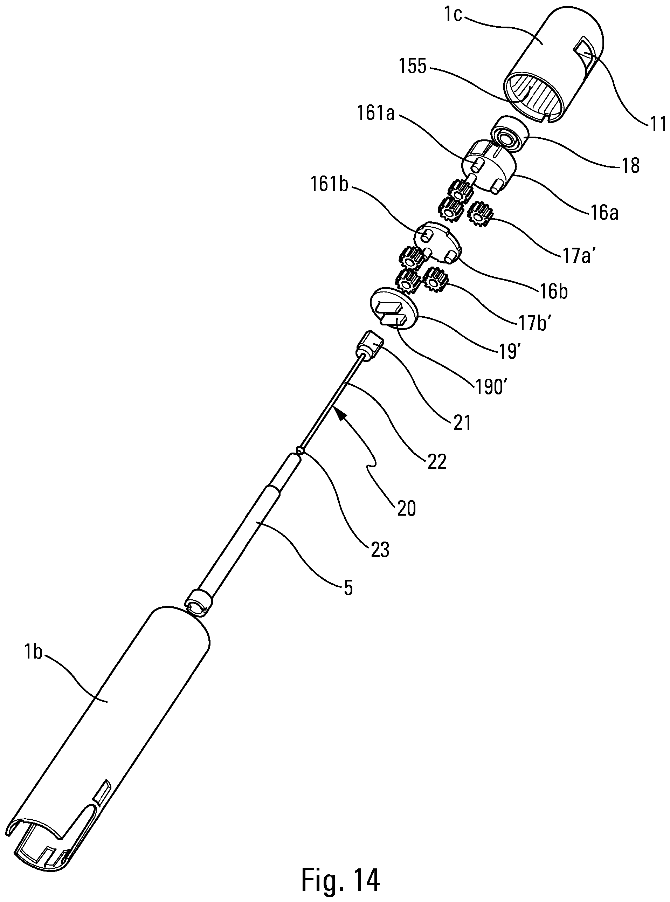

La figure 14 illustre une vue schématique en perspective éclatée de ce système retardateur selon le second mode de réalisation. Celui-ci comprend le corps supérieur 1c, un ressort retardateur 18, de préférence réalisé sous la forme d'un ressort à spirale, au moins un planétaire 16a, 16b, chacun avec au moins un satellite 17a', 17b', un déclencheur 19', une clé de verrouillage 20, la tige de piston 5 et le corps intermédiaire 1b.

Chaque planétaire 16a, 16b associé à ses satellites 17a', 17b' forme un étage du système retardateur. Dans l'exemple représenté sur les figures 13 à 19, il y a deux étages empilés axialement, avec un premier planétaire 16a et un second planétaire 16b, mais on peut prévoir un nombre quelconque d'étages, par exemple un seul étage ou plus de deux étages.

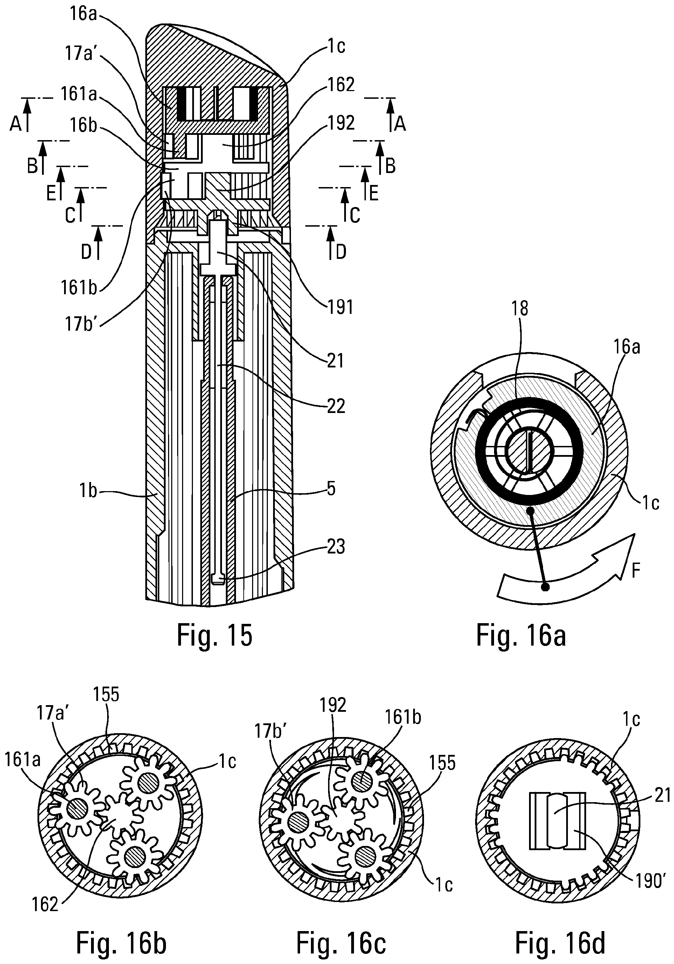

Le ressort à spirale 18 est fixé d'une part au premier planétaire 16a et d'autre part au corps supérieur 1c, comme visible dans la figure 16a. En variante, ledit ressort à spirale pourrait être fixé à un autre planétaire, par exemple le second planétaire 16b dans l'exemple représenté, ou au déclencheur 19'. De plus, le ressort à spirale pourrait être fixé à une autre partie du corps 1, par exemple le corps intermédiaire 1b, ou à tout élément fixé audit corps 1.

Dans l'exemple représenté, le premier planétaire 16a forme également un élément d'indication visuel du dispositif indicateur. En variante, cet élément indicateur pourrait être formé par un autre planétaire, par exemple le second planétaire 16b dans l'exemple représenté, ou par le déclencheur.

Chaque planétaire 16a, 16b comporte une partie en forme de disque sur laquelle sont formées d'un côté une ou plusieurs tige(s) de support de satellites 161a, 161b qui reçoivent chacune de manière rotative un satellite 17a', 17b'. Dans l'exemple représenté, il y a trois satellites 17a', 17b' à chaque étage, de sorte qu'il y a trois tiges 161a, 161b sur chaque planétaire 16a, 16b. Un nombre quelconque de satellites est toutefois possible.

Le second planétaire 16b comporte du côté axial opposé aux tiges de support 161b, un axe central 162 pourvu d'un engrenage, et coopérant avec les satellites 17a' du premier planétaire 16a.

Ainsi, le système retardateur utilise le principe des trains épicycloïdaux. Chaque étage de ce système permet de démultiplier et/ou ralentir les rotations de l'étage précédent.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un profil correspondant 12 du corps intermédiaire 1b et avec un profil correspondant 190' du déclencheur 19', de sorte que celui-ci est bloqué en rotation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit profil 190' du déclencheur 19', de sorte que ledit déclencheur 19' n'est plus bloqué en rotation par ladite clé 20.

Du côté axial opposé audit profil 190', le déclencheur comporte un axe central 192 pourvu d'un engrenage, et coopérant avec les satellites 17b' du second planétaire 16b.

S'il n'y avait qu'un seul étage au lieu des deux représentés, il n'y aurait pas le second planétaire 16b, et ce seraient les satellites 17a' du premier planétaire 16a qui coopéreraient directement avec l'axe central 192 du déclencheur 19'. De même, s'il y avait plus de deux étages, il aurait au moins un planétaire supplémentaire entre le second planétaire 16b et le déclencheur 19'.

Le corps supérieur 1c comporte un engrenage 155 sur sa surface interne latérale, comme clairement visible sur les figures 16b et 16c. Cet engrenage interne 155 du corps supérieur 1c coopère avec les satellites 17a', 17b' qui sont assemblés sur les planétaires 16a, 16b.

Le ressort à spirale 18 sollicite le premier planétaire en rotation dans le sens de la flèche F sur la figure 16a. Cette rotation est transmise via les satellites 17a' à l'axe central 162 du second planétaire 16b, et de là, via les satellites 17b' à l'axe central 192 du déclencheur 19. Tant que le déclencheur 19' est bloqué par ladite clé de verrouillage 20, le système retardateur est donc également bloqué.

Lorsque le système retardateur est déclenché, le ressort 18 sollicite en rotation le premier planétaire 16a. Les satellites 17a' sont donc entrainés en rotation du fait qu'il sont engrenés avec l'engrenage 155 du corps supérieur 1c. Cette rotation des satellites 17a' fait donc tourner l'axe central 162 du second planétaire 16b, et la même opération se répète avec ledit second planétaire 16b. La vitesse de rotation du premier planétaire 16a est donc inférieure à celle dudit second planétaire 16b. Chaque étage supplémentaire du train épicycloïdal formant le retardateur va davantage démultiplier ces rotations, et donc davantage ralentir la rotation du premier planétaire 16a. Ainsi, avec deux étages comme représenté sur les figures, on peut ramener à un seul tour la rotation du premier planétaire 16a alors que le déclencheur 19' réalisera simultanément environ vingt tours.

Selon le nombre d'étages et/ou selon de nombre de satellites et/ou selon la forme des planétaires et/ou selon les dimensions des engrenages en jeu, on peut régler assez précisément le temps entre le moment où le système retardateur est déclenché, en fin d'injection, et le moment où le premier planétaire 16a aura réalisé sa rotation prédéfinie pour réaliser l'indication, et notamment indiquer dans la fenêtre de l'indicateur que l'autoinjecteur peut être retiré du site d'injection. La fin d'actionnement du dispositif indicateur est donc retardée par rapport à la fin de l'injection, ce qui permet au produit injecté de se diffuser dans le site d'injection pendant cette durée de retard.

Le rapport des vitesses peut varier grandement, c'est-à-dire que le système peut être utilisé pour ralentir le premier planétaire 16a (rapport des vitesses supérieur à 1) ou pour ralentir le déclencheur 19' (rapport des vitesses inférieur à 1), par exemple si c'est le déclencheur qui forme l'élément indicateur du dispositif indicateur.

En variantes aux satellites à engrenages coopérant avec l'engrenage 155 du corps supérieur 1c, on peut aussi envisager une transmission différente, par exemple par friction.

Selon l'invention, on prévoit des moyens pour générer un bruit pendant la durée d'actionnement dudit système retardateur.

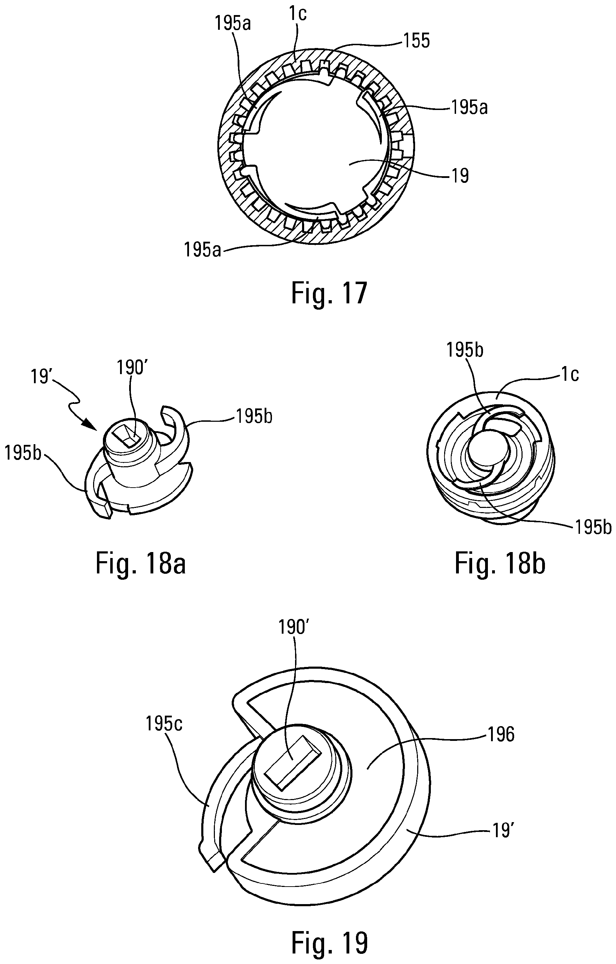

La figure 17 illustre un déclencheur 19' comportant trois pattes flexibles 195a qui glissent sur l'engrenage 155 du corps supérieur 1c, pour générer un bruit continu pendant la rotation dudit déclencheur 19'. Ceci fournit une indication sonore du fonctionnement du système retardateur: lorsque le bruit s'arrête, l'actionnement du système retardateur est terminé, et l'utilisateur peut retirer l'autoinjecteur du site d'injection. Un freinage par frottements peut aussi être fourni par ces pattes flexibles 195a.

Les figures 18a et 18b illustrent une autre variante, dans laquelle le déclencheur 19' comporte deux pattes flexibles 195b, avec les extrémités de ces pattes générant un bruit pendant toute la rotation dudit déclencheur 19', par exemple sur un profil approprié du corps supérieur. Bien entendu, ces pattes 195b peuvent simultanément aussi fournir un freinage de la rotation.

La figure 19 montre encore une autre variante, dans laquelle le déclencheur 19' comporte une seule patte flexible 195c, qui génère du bruit pendant la rotation, et qui peut aussi freiner cette rotation. Dans cette variante, le déclencheur 19' peut comporter une masse d'inertie 196, qui favorise le freinage.

Il est entendu que la ou les pattes flexibles 195a, 195b, 195c décrites ci-dessus pourraient aussi s'appliquer à un planétaire 16a, 16b plutôt qu'au déclencheur 19'.

Le troisième mode de réalisation des figures 20 à 23 exploite le phénomène de cisaillement de fluide pour générer ledit retard entre la fin d'actionnement du dispositif indicateur et la fin de l'injection, et utilise un réservoir 16', un organe de cisaillement 19" disposé dans ledit réservoir 16', et un fluide disposé dans ledit réservoir 16', autour dudit organe de cisaillement 19".

Dans l'exemple représenté, l'organe de cisaillement 19" est fixe en rotation par rapport au corps 1, et le réservoir 16' est mobile en rotation par rapport audit corps 1. L'inverse est toutefois aussi envisageable.

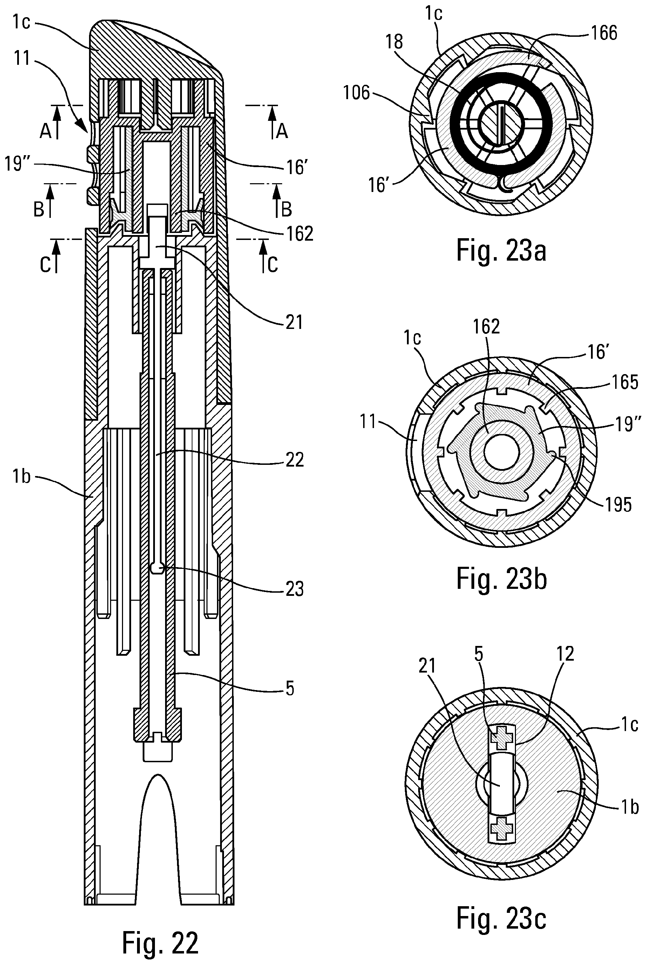

Un fluide est disposé dans ledit réservoir 16', autour dudit organe de cisaillement 19". Avantageusement, le réservoir 16' comporte des projections 165 sur sa surface interne et l'organe de cisaillement 19" comporte des projections 195 sur sa surface externe. Ces projections 165, 195 génèrent des restrictions à l'écoulement du fluide. Une rotation dudit réservoir 16' par rapport audit organe de cisaillement 19" implique donc un cisaillement du fluide, en particulier lorsqu'une projection 165 du réservoir 16' se trouve face à une projection 195 de l'organe de cisaillement 19", comme visible sur la figure 23b.

Le terme "cisaillement de fluide" est un phénomène de viscosité dynamique. La viscosité dynamique correspond à la contrainte de cisaillement qui accompagne l'existence d'un gradient de vitesse d'écoulement dans la matière. Lorsque la viscosité augmente, la capacité du fluide à s'écouler diminue.

On exploite également le phénomène de couche limite, qui est lié à la viscosité dynamique. La couche limite est la zone d'interface entre un corps et le fluide environnant lors d'un mouvement relatif entre les deux, conséquence de sa viscosité. Lorsqu'un fluide s'écoule le long d'une paroi supposée fixe, les vitesses sur la paroi sont nulles alors qu'à l'infini (c'est-à-dire loin de l'obstacle) elles sont égales à la vitesse de l'écoulement non perturbé. La loi de variation dépend de la viscosité du fluide qui induit un frottement entre les couches voisines : la couche la plus lente tend à freiner la couche la plus rapide qui, en retour, tend à l'accélérer. Dans ces conditions, une forte viscosité égalise au maximum les vitesses. Au contraire, si le fluide est peu visqueux, les différentes couches sont beaucoup plus indépendantes : la vitesse à l'infini se maintient jusqu'à une courte distance de l'obstacle et il y a une variation plus forte des vitesses dans la petite épaisseur de la couche limite.

Selon la viscosité du fluide contenu dans le réservoir 16' et/ou selon la forme et/ou les dimensions des profils 165, 195 du réservoir 16' et de l'organe de cisaillement 19", on peut régler assez précisément ledit freinage et donc le temps entre le moment où le système retardateur est déclenché, en fin d'injection, et le moment où le réservoir 16' aura réalisé sa rotation prédéfinie pour réaliser l'indication sonore et visuelle, et notamment générer un bruit continu pendant sa rotation et indiquer en fin de rotation dans la fenêtre de l'indicateur que l'autoinjecteur peut être retiré du site d'injection. La fin d'actionnement du dispositif indicateur est donc retardée par rapport à la fin de l'injection, ce qui permet au produit injecté de se diffuser dans le site d'injection pendant cette durée de retard.

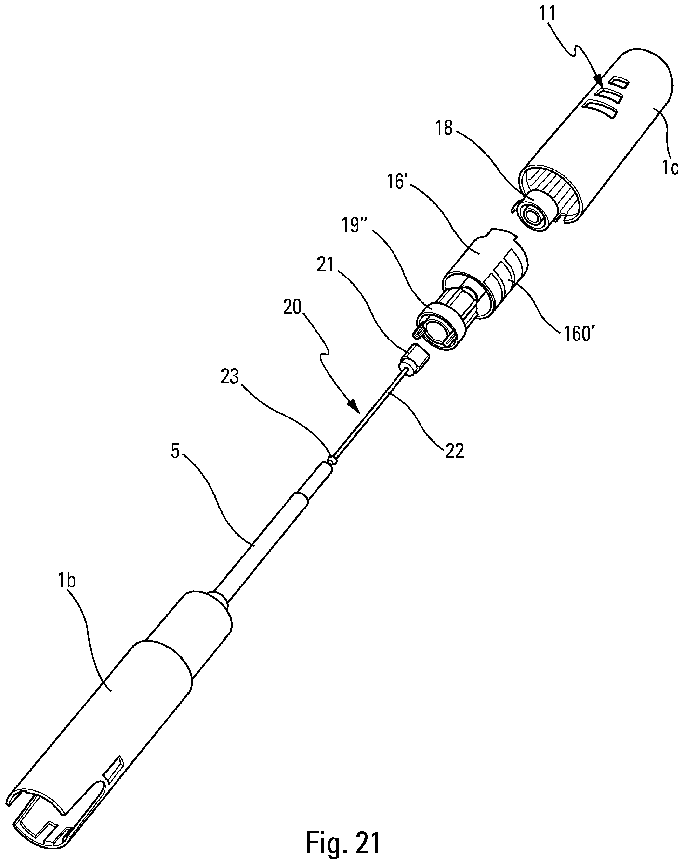

La figure 21 illustre une vue schématique en perspective éclatée de ce système retardateur selon le troisième mode de réalisation. Celui-ci comprend le corps supérieur 1c, un ressort retardateur 18, de préférence réalisé sous la forme d'un ressort à spirale, le réservoir 16' contenant un fluide approprié, l'organe de cisaillement 19" disposé dans ledit réservoir 16', une clé de verrouillage 20, la tige de piston 5 et le corps intermédiaire 1b.

Dans l'exemple représenté, le réservoir 16' forme également un élément d'indication visuelle du dispositif indicateur. Avantageusement, ledit réservoir 16' peut comporter un affichage 160' approprié pour venir indiquer la fin d'utilisation de l'autoinjecteur dans une ou plusieurs fenêtres du corps 1, notamment du corps supérieur 1c. Dans l'exemple représenté sur les figures 20 à 23, le corps 1 comporte trois fenêtres 11 qui affichent la progression de l'actionnement du système retardateur. La figure 21 illustre ce marquage 160 réalisé sur la surface externe dudit réservoir 16.

Le ressort à spirale 18 est fixé d'une part au corps supérieur 1c et d'autre part au réservoir 16', comme visible notamment dans la figure 23a. En variante, le ressort à spirale pourrait être fixé à une autre partie du corps 1, par exemple le corps intermédiaire 1b, ou à tout élément fixé audit corps 1. En variante, ledit ressort à spirale pourrait être fixé à l'organe de cisaillement 19", auquel cas c'est le réservoir 16' qui serait fixe en rotation par rapport au corps 1. Dans cette variante, cet organe de cisaillement mobile en rotation pourrait former l'élément indicateur du dispositif indicateur visuel, sonore et/ou tactile.

La clé de verrouillage 20 comporte une tête 21 adaptée à coopérer avec le système retardateur, une tige longitudinale 22, et en embout 23 adapté à coopérer avec la tige de piston 5.

En position avant déclenchement du système retardateur, la tête 21 de la clé de verrouillage 20 est en position de blocage dans laquelle elle coopère avec un profil correspondant 12 du corps intermédiaire 1b et avec un profil correspondant 161 du réservoir 16', de sorte que celui-ci est bloqué en rotation par ladite clé. Lorsque la tige de piston 5 arrive vers sa position de fin d'injection, elle va coopérer avec l'embout 23 de la clé de verrouillage 20, et ainsi tirer ladite clé axialement vers le bas. De ce fait, la tête 21 de ladite clé de verrouillage 20 sort axialement dudit profil 161 du réservoir 16', de sorte que ledit réservoir 16' n'est plus bloqué en rotation par ladite clé 20. Avantageusement, ledit profil 161 du réservoir est formé sur un manchon interne 162 du réservoir 16', disposé à l'intérieur dudit organe de cisaillement 19", comme visible notamment sur la figure 22.

Le ressort à spirale 18 sollicite le réservoir 16' en rotation. Tant que le réservoir 16' est bloqué par ladite clé de verrouillage 20, le système de retardement est donc également bloqué.

Lorsque le système retardateur est déclenché, le ressort 18 sollicite en rotation le réservoir 16'. Celui-ci subit un couple de freinage dû au cisaillement du fluide contenu entre la paroi du réservoir 16' et l'organe de cisaillement 19". La rotation du réservoir 16' est donc freinée par ledit fluide.

Selon l'invention, le réservoir 16' comporte au moins une patte flexible 166, visible sur la figure 23a, dont l'extrémité génère un bruit pendant toute la rotation dudit réservoir 16', par exemple sur des profils 106 appropriés du corps supérieur 1c, et fournissant ainsi une indication sonore du fonctionnement du système retardateur: lorsque le bruit s'arrête, l'actionnement du système retardateur et celui du dispositif indicateur sont terminés, et l'utilisateur peut retirer l'autoinjecteur du site d'injection. Bien entendu, cette patte 166 peut simultanément aussi fournir un freinage supplémentaire de la rotation. En variante, on peut prévoir plusieurs pattes flexibles 166, par exemple deux.

Dans le troisième mode de réalisation décrit ci-dessus, le fluide utilisé dans le système retardateur peut être de tout type approprié, par exemple une graisse.

Une phase complète d'actionnement de l'autoinjecteur va être décrite ci-après, qui s'applique aux trois modes de réalisation décrits précédemment.

Lorsque l'utilisateur veut utiliser l'autoinjecteur, il prend le dispositif, par exemple au niveau du corps 1 et il appuie le manchon actionneur 10, qui au repos, dans sa première position projetée, fait saillie hors du corps inférieur 1, contre la partie du corps où il veut réaliser l'injection. La pression exercée par l'utilisateur sur le manchon actionneur 10 provoque le coulissement de celui-ci vers l'intérieur du corps 1, ce qui découvre l'aiguille et donc son piquage en raison de la pression exercée par l'utilisateur sur l'autoinjecteur.

Lorsque le manchon actionneur 10 atteint sa position d'actionnement, qui est sa position d'extrémité à l'intérieur du corps 1, il provoque le déclenchement de la phase d'injection. On constate que la tige de piston 5 coulisse à l'intérieur de la seringue A en poussant le piston P de celle-ci sous l'effet du ressort d'injection 8. Le produit est donc distribué.

A la fin de l'injection, le dispositif indicateur est déclenché, et le système retardateur agit de sorte que le dispositif indicateur ne sera actionné complètement qu'après un temps de retard prédéterminé, générant un bruit continu entre la fin d'injection et la fin d'actionnement du dispositif indicateur.

Après indication de la fin d'utilisation, lorsque l'utilisateur peut retirer l'autoinjecteur du site d'injection, le manchon actionneur 10 est à nouveau déplacé hors du corps 1 vers la position de fin d'utilisation, qui est sa seconde position projetée, sous l'effet du ressort du manchon actionneur, avec un verrouillage dudit manchon actionneur 10, ce qui garantit une sécurité absolue pour l'utilisateur et évite tout risque de blessures avec l'aiguille après utilisation du dispositif.

Il est à noter que les première et seconde positions projetées du manchon actionneur, qui, dans l'exemple représenté, sont des positions différentes, pourraient éventuellement être identiques.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à plusieurs modes de réalisation avantageux, il est entendu que ceux-ci ne sont pas limitatifs. En particulier, le manchon actionneur et/ou la serrure d'injection et/ou le dispositif retardateur et/ou le dispositif indicateur pourrai(en)t être réalisés différemment. Le piquage de l'aiguille et/ou une rétractation de l'aiguille après injection pourrait être commandé par un ou plusieurs bouton(s). D'autres modifications sont également possibles pour l'homme du métier sans sortir du cadre de la présente invention tel que défini par les revendications annexées.

## Revendications

1. Autoinjecteur comportant:
- un corps (1) recevant un réservoir (S), ledit réservoir (S) contenant du produit fluide et comportant un piston (P), tel qu'une seringue pré-remplie,
- une tige de piston (5) adaptée à coopérer avec le piston (P) dudit réservoir (S), ladite tige de piston (5) étant déplaçable par un ressort d'injection (8) entre une position chargée et une position d'injection dans laquelle ladite tige de piston (5) a déplacé le piston (P) du réservoir (S) pour injecter le produit fluide dans un site d'injection,
- un dispositif indicateur pour indiquer à l'utilisateur qu'il peut retirer ledit autoinjecteur dudit site d'injection,
ledit autoinjecteur comportant un système retardateur pour retarder la fin d'actionnement dudit dispositif indicateur par rapport à la fin de l'injection, **caractérisé en ce que** ledit dispositif indicateur génère un bruit en continu pendant son actionnement, ledit bruit étant généré jusqu'à la fin d'actionnement dudit dispositif indicateur, ledit dispositif indicateur et/ou ledit système retardateur comprenant un élément mobile en rotation (19; 19'; 16') par rapport audit corps (1), ledit bruit continu étant généré pendant cette rotation notamment au moyen d'une ou plusieurs patte(s) flexible(s) formée(s) soit sur ledit élément mobile en rotation, soit sur ledit corps, et coopérant avec des profils adaptés formés sur l'autre parmi ledit élément mobile et ledit corps.

2. Autoinjecteur selon la revendication 1, dans lequel ledit dispositif indicateur comprend un capot (1000), le corps (1), une bande indicatrice (160), un ressort indicateur (18), une roue motrice (19), une roue d'enroulement (17a) et une roue de déroulement (17b).

3. Autoinjecteur selon la revendication 2, dans lequel ledit ressort indicateur (18) est réalisé sous la forme d'un ressort à spirale fixé d'une part à ladite roue motrice (19) et d'autre part audit capot (1000).

4. Autoinjecteur selon la revendication 2 ou 3, dans lequel ladite roue motrice (19) comporte un engrenage (195) qui coopère avec un engrenage (175) de la roue d'enroulement (17a), de sorte qu'une rotation de ladite roue motrice (19) entraine une rotation de ladite roue d'enroulement (17a), ce qui provoque l'enroulement de ladite bande indicatrice (160) sur ladite roue d'enroulement (17a).

5. Autoinjecteur selon l'une quelconque des revendications 2 à 4, dans lequel ladite clé de verrouillage (20) comporte une tête (21), une tige longitudinale (22), et en embout (23) adapté à coopérer avec la tige de piston (5).

6. Autoinjecteur selon la revendication 5, dans lequel, avant déclenchement du dispositif indicateur, la tête (21) de la clé de verrouillage (20) est en position de blocage dans laquelle elle coopère avec un profil correspondant (190) de la roue motrice (19), de sorte que ladite roue motrice (19) est bloquée en rotation par rapport audit corps (1) et audit capot (1000) par ladite clé de verrouillage (20).

7. Autoinjecteur selon la revendication 6, dans lequel lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ladite roue motrice (19) n'est alors plus bloquée en rotation par ladite clé de verrouillage (20).

8. Autoinjecteur selon l'une quelconque des revendications 2 à 7, dans lequel ledit corps (1) comporte au moins une patte flexible (1100) adaptée à coopérer avec ladite roue motrice (19) pour générer un bruit pendant la rotation de ladite roue motrice (19).

9. Autoinjecteur selon la revendication 1, dans lequel ledit système retardateur comporte un train épicycloïdal ayant au moins un, avantageusement deux, étage(s), ledit train épicycloïdal comportant un ressort retardateur (18), au moins un planétaire (16a, 16b), chacun comportant au moins un satellite (17a', 17b'), un déclencheur (19'), et une clé de verrouillage (20) pour bloquer ledit déclencheur (19') en rotation jusqu'en fin d'injection.

10. Autoinjecteur selon la revendication 9, dans lequel ledit ressort retardateur (18) est réalisé sous la forme d'un ressort à spirale fixé d'une part à un planétaire (16a, 16b) ou au déclencheur (19') et d'autre part au corps (1).

11. Autoinjecteur selon la revendication 9 ou 10, dans lequel chaque satellite (17a', 17b') d'un planétaire (16a, 16b) coopère d'une part avec ledit corps (1) et d'autre part soit avec un autre planétaire (16b, 16a), soit avec ledit déclencheur (19').

12. Autoinjecteur selon la revendication 11, dans lequel ledit corps (1) comporte un engrenage (155) sur sa surface interne latérale, ledit engrenage (155) coopérant avec au moins un satellite (17a', 17b').

13. Autoinjecteur selon la revendication 11 ou 12, dans lequel ledit déclencheur (19') comporte un axe central (192) pourvu d'un engrenage coopérant avec au moins un satellite (17a', 17b').

14. Autoinjecteur selon l'une quelconque des revendications 9 à 13, dans lequel ledit train épicycloïdal comporte deux planétaires (16a, 16b), chacun comportant trois satellites (17a', 17b').

15. Autoinjecteur selon l'une quelconque des revendications 9 à 14, dans lequel ladite clé de verrouillage (20) comporte une tête (21), une tige longitudinale (22), et en embout (23) adapté à coopérer avec la tige de piston (5).

16. Autoinjecteur selon la revendication 15, dans lequel, avant déclenchement du système retardateur, la tête (21) de la clé de verrouillage (20) est en position de blocage dans laquelle elle coopère avec un profil correspondant (12) du corps (1) et avec un profil correspondant (190') du déclencheur (19'), de sorte que ledit déclencheur (19') est bloqué en rotation par rapport audit corps (1) par ladite clé de verrouillage (20).

17. Autoinjecteur selon la revendication 16, dans lequel lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ledit déclencheur (19') n'est alors plus bloqué en rotation par ladite clé de verrouillage (20).

18. Autoinjecteur selon l'une quelconque des revendications 9 à 17, dans lequel ledit déclencheur (19') comporte au moins une patte flexible (195a; 195b; 195c) adaptée à coopérer avec ledit corps (1) pour générer un bruit pendant la rotation dudit déclencheur (19').

19. Autoinjecteur selon la revendication 1, dans lequel ledit système retardateur comporte un réservoir (16'), un organe de cisaillement (19") disposé dans ledit réservoir (16') et un fluide disposé dans ledit réservoir (16') autour dudit organe de cisaillement (19"), l'un parmi ledit réservoir (16') et ledit organe de cisaillement (19") étant monté rotatif dans ledit corps (1) et l'autre parmi ledit réservoir (16') et ledit organe de cisaillement (19") étant fixe en rotation, la rotation de l'un par rapport à l'autre étant freinée par cisaillement dudit fluide contenu dans ledit réservoir (16').

20. Autoinjecteur selon la revendication 19, dans lequel ledit réservoir (16') est monté rotatif dans ledit corps (1) et ledit organe de cisaillement (19") est fixe en rotation.

21. Autoinjecteur selon la revendication 19 ou 20, dans lequel ledit réservoir (16') comporte des projections (165) sur sa surface interne et ledit organe de cisaillement (19") comporte des projections (195) sur sa surface externe, lesdites projections (165, 195) générant des restrictions à l'écoulement du fluide.

22. Autoinjecteur selon l'une quelconque des revendications 19 à 21, dans lequel ledit système retardateur comprend ledit réservoir (16') contenant ledit fluide, ledit organe de cisaillement (19"), un ressort retardateur (18), une clé de verrouillage (20) et ladite tige de piston (5).

23. Autoinjecteur selon la revendication 22, dans lequel ladite clé de verrouillage (20) comporte une tête (21), une tige longitudinale (22) et en embout (23) adapté à coopérer avec la tige de piston (5).

24. Autoinjecteur selon la revendication 23, dans lequel, avant déclenchement du système retardateur, la tête (21) de la clé de verrouillage (20) est en position de blocage dans laquelle elle coopère avec un profil correspondant (12) du corps (1) et avec un profil correspondant (161) dudit réservoir (16'), de sorte que ledit réservoir (16') est bloqué en rotation par rapport audit corps (1) par ladite clé de verrouillage (20).

25. Autoinjecteur selon la revendication 24, dans lequel, lorsque la tige de piston (5) arrive vers sa position de fin d'injection, elle coopère avec l'embout (23) de la clé de verrouillage (20) pour tirer ladite clé de verrouillage (20) axialement vers le bas hors de sa position de blocage, de sorte que ledit réservoir (16') n'est alors plus bloqué en rotation par ladite clé de verrouillage (20).

26. Autoinjecteur selon l'une quelconque des revendications 22 à 25, dans lequel ledit ressort retardateur (18) est réalisé sous la forme d'un ressort à spirale fixé d'une part audit réservoir (16') ou audit organe de cisaillement (19") et d'autre part audit corps (1).

27. Autoinjecteur selon la revendication 26, dans lequel ledit réservoir (16') comporte au moins une patte flexible (166) coopérant avec une pluralité de profils (106) dudit corps (1), pour générer un bruit continu pendant l'actionnement dudit système retardateur.

28. Autoinjecteur selon l'une quelconque des revendications précédentes, dans lequel ledit autoinjecteur comporte un manchon actionneur (10) comportant une extrémité de contact (101) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur.

29. Autoinjecteur selon l'une quelconque des revendications précédentes, dans lequel ledit réservoir (S) comporte une aiguille (A) à travers laquelle ledit produit fluide est injecté dans ledit site d'injection.

30. Autoinjecteur selon l'une quelconque des revendications précédentes, dans lequel l'un parmi ledit élément mobile en rotation (19; 19'; 16') et ledit corps (1) comporte au moins une patte flexible (1100; 195a, 195b, 195c; 166) coopérant avec des profils adaptés formés sur l'autre parmi ledit élément mobile en rotation (19; 19'; 16') et ledit corps (1) pour générer ledit bruit.

31. Autoinjecteur selon la revendication 30, dans lequel les formes et/ou dimensions de ladite au moins une patte flexible et/ou desdits profils adaptés sont variables, pour générer un bruit variable.

## Patentansprüche

1. Autoinjektor, folgendes umfassend:
- einen Körper (1) zur Aufnahme eines Behälters (S), welcher ein Flüssigprodukt enthält und einen Kolben (P) aufweist, zum Beispiel einer vorgefüllten Spritze,
- eine Kolbenstange (5), die geeignet ist, mit dem Kolben (P) des Behälters (S) zusammenzuwirken, wobei die Kolbenstange (5) durch eine Injektionsfeder (8) verfahrbar ist zwischen einer geladenen Stellung und einer Injektionsstellung, in welcher die Kolbenstange (5) den Kolben (P) des Behälters (S) verfahren hat, um das Flüssigprodukt in eine Injektionsstelle zu injizieren,
- eine Anzeigevorrichtung, die dem Benutzer anzeigt, dass der Autoinjektor von der Injektionsstelle entfernt werden kann,
wobei der Autoinjektor ein Verzögerungssystem zum Verzögern des Endes der Betätigung der Anzeigevorrichtung in Bezug auf das Ende der Injektion umfasst, **dadurch gekennzeichnet, dass** die Anzeigevorrichtung während ihrer Betätigung einen Dauerton erzeugt, wobei der Dauerton bis zum Ende der Betätigung der Anzeigevorrichtung erzeugt wird, wobei die Anzeigevorrichtung und/oder das Verzögerungssystem ein in Bezug auf den Körper (1) drehbewegliches Element (19; 19'; 16') umfasst; wobei der Dauerton während dieser Drehung erzeugt wird, insbesondere mittels einer oder mehrerer flexibler Laschen, die entweder an dem drehbeweglichen Element oder an dem Körper ausgebildet sind und mit passenden Profilen zusammenwirken, die an dem jeweils anderen Teil, als dem beweglichen Element bzw. dem Körper ausgebildet sind.

2. Autoinjektor nach Anspruch 1, bei dem die Anzeigevorrichtung eine Abdeckkappe (1000), den Körper (1), einen Anzeigestreifen (160), eine Anzeigefeder (18), ein Antriebsrad (19), ein Aufwickelrad (17a) und ein Abwickelrad (17b) umfasst.

3. Autoinjektor nach Anspruch 2, bei dem die Anzeigefeder (18) in Form einer Spiralfeder ausgeführt ist, die einerseits an dem Antriebsrad (19) und andererseits an der Abdeckkappe (1000) befestigt ist.

4. Autoinjektor nach Anspruch 2 oder 3, bei dem das Antriebsrad (19) ein Zahnrad (195) aufweist, das mit einem Zahnrad (175) des Aufwickelrades (17a) derart zusammenwirkt, dass eine Drehung des Antriebsrades (19) eine Drehung des Aufwickelrades (17a) bewirkt, wodurch der Anzeigestreifen (160) auf das Aufwickelrad (17a) aufgewickelt wird.

5. Autoinjektor nach einem der Ansprüche 2 bis 4, bei dem der Verriegelungsschlüssel (20) einen Kopf (21), eine Längsstange (22) und eine Spitze (23) aufweist, die geeignet ist, mit der Kolbenstange (5) zusammenzuwirken.

6. Autoinjektor nach Anspruch 5, bei dem sich vor dem Auslösen der Anzeigevorrichtung der Kopf (21) des Verriegelungsschlüssels (20) in einer Sperrstellung befindet, in der er mit einem entsprechenden Profil (190) des Antriebsrades (19) derart zusammenwirkt, dass der Verriegelungsschlüssel (20) das Antriebsrad (19) bezüglich seiner Drehung relativ zu dem Körper (1) und der Abdeckkappe (1000) sperrt.

7. Autoinjektor nach Anspruch 6, bei dem die Kolbenstange (5) bei Erreichen ihrer Injektionsendstellung mit der Spitze (23) des Verriegelungsschlüssels (20) zusammenwirkt, um den Verriegelungsschlüssel (20) axial nach unten aus seiner Sperrstellung zu ziehen, so dass der Verriegelungsschlüssel (20) das Antriebsrad (19) nicht mehr bezüglich seiner Drehung blockiert.

8. Autoinjektor nach einem der Ansprüche 2 bis 7, bei dem der Körper (1) mindestens eine flexible Lasche (1100) aufweist, die geeignet ist, mit dem Antriebsrad (19) zusammenzuwirken, um während der Drehung des Antriebsrades (19) einen Ton zu erzeugen.

9. Autoinjektor nach Anspruch 1, bei dem das Verzögerungssystem ein Planetengetriebe mit mindestens einer, vorzugsweise zwei, Stufe(n) umfasst, wobei das Planetengetriebe eine Verzögerungsfeder (18), mindestens ein Sonnenrad (16a, 16b), von denen jedes mindestens ein Planetenrad (17a', 17b') umfasst, einen Auslöser (19') und einen Verriegelungsschlüssel (20) zum Sperren des Auslösers (19') gegen Drehung bis zum Ende der Injektion umfasst.

10. Autoinjektor nach Anspruch 9, bei dem die Verzögerungsfeder (18) als Spiralfeder ausgebildet ist, die einerseits an einem Sonnenrad (16a, 16b) oder an dem Auslöser (19') und andererseits an dem Körper (1) befestigt ist.

11. Autoinjektor nach Anspruch 9 oder 10, bei dem jedes Planetenrad (17a', 17b') eines Sonnenrades (16a, 16b) einerseits mit dem Körper (1) und andererseits entweder mit einem anderen Sonnenrad (16b, 16a) oder mit dem Auslöser (19') zusammenwirkt.

12. Autoinjektor nach Anspruch 11, bei dem der Körper (1) an seiner seitlichen Innenfläche ein Zahnrad (155) aufweist, wobei das Zahnrad (155) mit mindestens einem Planetenrad (17a', 17b') zusammenwirkt.

13. Autoinjektor nach Anspruch 11 oder 12, bei dem der Auslöser (19') eine Mittelachse (192) aufweist, die ein Zahnrad trägt, das mit mindestens einem Planetenrad (17a', 17b') zusammenwirkt.

14. Autoinjektor nach einem der Ansprüche 9 bis 13, bei dem das Planetengetriebe zwei Sonnenräder (16a, 16b) mit jeweils drei Planetenrädern (17a', 17b') umfasst.

15. Autoinjektor nach einem der Ansprüche 9 bis 14, bei dem der Verriegelungsschlüssel (20) einen Kopf (21), eine Längsstange (22) und eine Spitze (23) aufweist, die geeignet ist, mit der Kolbenstange (5) zusammenzuwirken.

16. Autoinjektor nach Anspruch 15, bei dem sich vor dem Auslösen des Verzögerungssystems der Kopf (21) des Verriegelungsschlüssels (20) in einer Sperrstellung befindet, in der er mit einem entsprechenden Profil (12) des Körpers (1) und mit einem entsprechenden Profil (190') des Auslösers (19') zusammenwirkt, so dass der Verriegelungsschlüssel (20) den Auslöser (19') bezüglich seiner Drehung relativ zum Körper (1) sperrt.

17. Autoinjektor nach Anspruch 16, bei dem die Kolbenstange (5) bei Erreichen ihrer Injektionsendstellung mit der Spitze (23) des Verriegelungsschlüssels (20) zusammenwirkt, um den Verriegelungsschlüssel (20) axial nach unten aus seiner Sperrstellung zu ziehen, so dass der Verriegelungsschlüssel (20) den Auslöser (19') nicht mehr bezüglich dessen Drehung sperrt.

18. Autoinjektor nach einem der Ansprüche 9 bis 17, bei dem der Auslöser (19') mindestens eine flexible Lasche (195a; 195b; 195c) aufweist, die geeignet ist, mit dem Körper (1) zusammenzuwirken, um während der Drehung des Auslösers (19') einen Ton zu erzeugen.

19. Autoinjektor nach Anspruch 1, bei dem das Verzögerungssystem einen Behälter (16'), ein in dem Behälter (16') angeordnetes Scherelement (19") und eine in dem Behälter (16') um das Scherelement (19") herum angeordnete Flüssigkeit umfasst, wobei entweder der Behälter (16') oder das Scherelement (19") drehbar in dem Körper (1) angeordnet ist und das jeweils andere Element, also der Behälter (16') bzw. das Scherelement (19"), drehfest angeordnet ist, wobei die Drehung des einen Elements in Bezug auf das andere durch das Scheren der in dem Behälter (16') enthaltenen Flüssigkeit gebremst wird.

20. Autoinjektor nach Anspruch 19, bei dem der Behälter (16') drehbar in dem Körper (1) angebracht ist und das Scherelement (19") drehfest angeordnet ist.

21. Autoinjektor nach Anspruch 19 oder 20, bei dem der Behälter (16') Vorsprünge (165) an seiner Innenfläche und das Scherelement (19") Vorsprünge (195) an seiner Außenfläche aufweist, wobei die Vorsprünge (165, 195) Strömungsbegrenzungen für die Flüssigkeit erzeugen.

22. Autoinjektor nach einem der Ansprüche 19 bis 21, bei dem das Verzögerungssystem den die Flüssigkeit enthaltenden Behälter (16'), das Scherelement (19"), eine Verzögerungsfeder (18), einen Verriegelungsschlüssel (20) und die Kolbenstange (5) umfasst.

23. Autoinjektor nach Anspruch 22, bei dem der Verriegelungsschlüssel (20) einen Kopf (21), eine Längsstange (22) und eine Spitze (23) aufweist, die geeignet ist, mit der Kolbenstange (5) zusammenzuwirken.

24. Autoinjektor nach Anspruch 23, bei dem sich vor dem Auslösen des Verzögerungssystems der Kopf (21) des Verriegelungsschlüssels (20) in einer Sperrstellung befindet, in der er mit einem entsprechenden Profil (12) des Körpers (1) und mit einem entsprechenden Profil (161) des Behälters (16') zusammenwirkt, so dass der Verriegelungsschlüssel (20) den Behälter (16') bezüglich einer Drehung relativ zum Körper (1) sperrt.

25. Autoinjektor nach Anspruch 24, bei dem die Kolbenstange (5) bei Erreichen ihrer Injektionsendstellung mit der Spitze (23) des Verriegelungsschlüssels (20) zusammenwirkt, um den Verriegelungsschlüssel (20) axial nach unten aus seiner Sperrstellung zu ziehen, so dass der Verriegelungsschlüssel (20) den Behälter (16') nicht mehr bezüglich dessen Drehung sperrt.

26. Autoinjektor nach einem der Ansprüche 22 bis 25, bei dem die Verzögerungsfeder (18) als Spiralfeder ausgebildet ist, die einerseits an dem Behälter (16') oder an dem Scherelement (19") und andererseits an dem Körper (1) befestigt ist.

27. Autoinjektor nach Anspruch 26, bei dem der Behälter (16') mindestens eine flexible Lasche (166) aufweist, die mit einer Vielzahl von Profilen (106) des Körpers (1) zusammenwirkt, um während der Betätigung des Verzögerungssystems einen Dauerton zu erzeugen.

28. Autoinjektor nach einem der vorstehenden Ansprüche, bei dem der Autoinjektor eine Betätigungshülse (10) mit einem Kontaktende (101) zum Kontaktieren des Körpers des Benutzers aufweist, wobei die Betätigungshülse (10) zumindest teilweise innerhalb des Körpers (1) verläuft und relativ zu dem Körper (1) verfahrbar ist zwischen vorstehenden Positionen, in denen die Betätigungshülse (10) zumindest teilweise aus dem Körper (1) herausragt, und einer Betätigungsposition, in der die Betätigungshülse (10) axial in Richtung zum Inneren des Körpers (1) hin verschoben ist, wobei sich die Betätigungshülse (10) vor Betätigung des Autoinjektors in einer ersten vorstehenden Position und nach Betätigung des Autoinjektors in einer zweiten vorstehenden Position befindet.

29. Autoinjektor nach einem der vorstehenden Ansprüche, bei dem der Behälter (S) eine Nadel (A) umfasst, über die die Injektion des Flüssigprodukts in die Injektionsstelle erfolgt.

30. Autoinjektor nach einem der vorstehenden Ansprüche, bei dem entweder das drehbewegliche Element (19; 19'; 16') oder der Körper (1) mindestens eine flexible Lasche (1100; 195a, 195b, 195c; 166) aufweist, die mit passenden Profilen zusammenwirkt, welche an dem jeweils anderen Teil, d.h. dem drehbeweglichen Element (19; 19'; 16') bzw. dem Körper (1) ausgebildet sind, um den Ton zu erzeugen.

31. Autoinjektor nach Anspruch 30, bei dem zur Erzeugung verschiedener Töne die mindestens eine flexible Lasche und/oder die entsprechenden Profile hinsichtlich Form und/oder Größe unterschiedlich ausführbar sind.

## Claims

1. An autoinjector comprising:
• a body (1) receiving a reservoir (S), said reservoir (S) containing fluid and including a piston (P), such as a pre-filled syringe,
• a piston rod (5) that is adapted to co-operate with the piston (P) of said reservoir (S), said piston rod (5) being movable by an injection spring (8) between a primed position and an injection position in which said piston rod (5) has moved the piston (P) of the reservoir (S) so as to inject the fluid into an injection site,
• an indicator device for indicating to the user that said autoinjector may be removed from said injection site,
said autoinjector including a retarding system so as to delay the end of actuating said indicator device relative to the end of injection, **characterized in that** said indicator device generates a continuous noise while it is being actuated, said noise being generated until the end of actuating said indicator device, said indicator device and/or said retarding system including a rotary element (19; 19'; 16') with respect to said body (1), said continuous noise being generated during said rotation, in particular by means of one or more flexible tabs formed either on said rotary element or on said body, and cooperating with corresponding profiles formed on the other of said rotary element and said body.

2. An autoinjector according to claim 1, wherein said indicator device comprises a cap (1000), the body (1), an indicator tape (160), an indicator spring (18), a drive wheel (19), a winder wheel (17a) and an unwinder wheel (17b).

3. An autoinjector according to claim 2, wherein said indicator spring (18) is made in the form of a spiral spring that is fastened firstly to said drive wheel (19) and secondly to said cap (1000).

4. An autoinjector according to claim 2 or claim 3, wherein said drive wheel (19) includes a gear (195) that co-operates with a gear (175) of the winder wheel (17a), such that turning said drive wheel (19) causes said winder wheel (17a) to turn, which causes said indicator tape (160) to be wound onto said winder wheel (17a).

5. An autoinjector according to any one of claims 2 to 4, wherein said locking key (20) comprises a head (21), a longitudinal rod (22), and an endpiece (23) that is adapted to co-operate with the piston rod (5).

6. An autoinjector according to claim 5, wherein, prior to triggering the indicator device, the head (21) of the locking key (20) is in its blocking position in which it co-operates with a corresponding profile (190) of the drive wheel (19), such that said drive wheel (19) is prevented from turning relative to said body (1) and to said cap (1000) by said locking key (20).

7. An autoinjector according to claim 6, wherein, when the piston rod (5) arrives towards its end-of-injection position, it co-operates with the endpiece (23) of the locking key (20) so as to pull said locking key (20) axially downwards out from its blocking position, such that said drive wheel (19) is thus no longer prevented from turning by said locking key (20).

8. An autoinjector according to any one of claims 2 to 7, wherein said body (1) includes at least one flexible tab (1100) that is adapted to co-operate with said drive wheel (19) so as to generate a noise while said drive wheel (19) is turning.

9. An autoinjector according to claim 1, wherein said retarding system comprises an epicyclic gear train having at least one, advantageously two stage(s), said epicyclic gear train comprising a retarding spring (18), at least one planet carrier (16a, 16b), each carrying at least one planet gear (17a', 17b'), a trigger (19'), and a locking key (20) so as to prevent said trigger (19') from turning until the end of injection.

10. An autoinjector according to claim 9, wherein said retarding spring (18) is made in the form of a spiral spring that is fastened firstly to a planet carrier (16a, 16b) or to the trigger (19') and secondly to the body (1).

11. An autoinjector according to claim 9 or claim 10, wherein each planet gear (17a', 17b') of a planet carrier (16a, 16b) co-operates firstly with said body (1) and secondly either with another planet carrier (16b, 16a), or with said trigger (19').

12. An autoinjector according to claim 11, wherein said body (1) includes a ring gear (155) on its inside surface, said ring gear (155) co-operating with at least one planet gear (17a', 17b').

13. An autoinjector according to claim 11 or claim 12, wherein said trigger (19') includes a central pin (192) that is provided with a sun gear that co-operates with at least one planet gear (17a', 17b').

14. An autoinjector according to any one of claims 9 to 13, wherein said epicyclic gear train comprises two planet carriers (16a, 16b), each carrying three planet gears (17a', 17b').

15. An autoinjector according to any one of claims 9 to 14, wherein said locking key (20) comprises a head (21), a longitudinal rod (22), and an endpiece (23) that is adapted to co-operate with the piston rod (5).

16. An autoinjector according to claim 15, wherein, prior to triggering the retarding system, the head (21) of the locking key (20) is in its blocking position in which it co-operates with a corresponding profile (12) of the body (1) and with a corresponding profile (190') of the trigger (19'), such that said trigger (19') is prevented from turning relative to said body (1) by said locking key (20).

17. An autoinjector according to claim 16, wherein when the piston rod (5) arrives towards its end-of-injection position, it co-operates with the endpiece (23) of the locking key (20) so as to pull said locking key (20) axially downwards out from its blocking position, such that said trigger (19') is thus no longer prevented from turning by said locking key (20).

18. An autoinjector according to any one of claims 9 to 17, wherein said trigger (19') includes at least one flexible tab (195a; 195b; 195c) that is adapted to co-operate with said body (1) so as to generate a noise while said trigger (19') is turning.

19. An autoinjector according to claim 1, wherein said retarding system comprises a dashpot (16'), a shear member (19") arranged in said dashpot (16'), and a fluid arranged in said dashpot (16') around said shear member (19"), one of said dashpot (16') and of said shear member (19") being rotatably mounted in said body (1), and the other one of said dashpot (16') and of said shear member (19") being stationary in rotation, the turning of one relative to the other being braked by shearing said fluid contained in said dashpot (16').

20. An autoinjector according to claim 19, wherein said dashpot (16') is rotatably mounted in said body (1), and said shear member (19") is stationary in rotation.

21. An autoinjector according to claim 19 or claim 20, wherein said dashpot (16') includes projections (165) on its inside surface and said shear member (19") includes projections (195) on its outer surface, said projections (165, 195) generating impediments to the flow of the fluid.

22. An autoinjector according to any one of claims 19 to 21, wherein said retarding system comprises said dashpot (16') containing said fluid, said shear member (19"), a retarding spring (18), a locking key (20) and said piston rod (5).

23. An autoinjector according to claim 22, wherein said locking key (20) comprises a head (21) a longitudinal rod (22), and an endpiece (23) that is adapted to co-operate with the piston rod (5).

24. An autoinjector according to claim 23, wherein, prior to triggering the retarding system, the head (21) of the locking key (20) is in its blocking position in which it co-operates with a corresponding profile (12) of the body (1) and with a corresponding profile (161) of said dashpot (16'), such that said dashpot (16') is prevented from turning relative to said body (1) by said locking key (20).

25. An autoinjector according to claim 24, wherein, when the piston rod (5) arrives towards its end-of-injection position, it co-operates with the endpiece (23) of the locking key (20) so as to pull said locking key (20) axially downwards out from its blocking position, such that said dashpot (16') is thus no longer prevented from turning by said locking key (20).

26. An autoinjector according to any one of claims 22 to 25, wherein said retarding spring (18) is made in the form of a spiral spring that is fastened firstly to said dashpot (16') or to said shear member (19") and secondly to said body (1).

27. An autoinjector according to claim 26, wherein said dashpot (16') includes at least one flexible tab (166) that co-operates with a plurality of profiles (106) of said body (1), so as to generate a continuous noise while said retarding system is being actuated.

28. An autoinjector according to any preceding claim, wherein said autoinjector includes an actuator sleeve (10) that includes a contact end (101) for coming into contact with the user's body, said actuator sleeve (10) extending inside said body (1) at least in part, and being movable relative to said body (1) between projecting positions, in which said actuator sleeve (10) projects out from said body (1) at least in part, and an actuated position in which said actuator sleeve (10) is moved axially into said body (1), said actuator sleeve (10) being in a first projecting position before actuation of the autoinjector, and in a second projecting position after actuation of the autoinjector.

29. An autoinjector according to any preceding claim, wherein said reservoir (S) includes a needle (A) through which said fluid is injected into said injection site.

30. An autoinjector according to any preceding claim, wherein one of said rotary element (19; 19'; 16') and of said body (1) includes at least one flexible tab (1100; 195a, 195b, 195c; 166) that co-operates with adapted profiles that are formed on the other one of said rotary element (19; 19'; 16') and of said body (1) so as to generate said noise.

31. An autoinjector according to claim 30, wherein the shapes and/or dimensions of said at least one flexible tab and/or of said adapted profiles vary so as to generate a noise that varies.
